(19) Europäisches Patentamt / European Patent Office / Office européen des brevets

(11) **EP 2 968 707 B1**

(12) **EUROPEAN PATENT SPECIFICATION**

(45) Date of publication and mention of the grant of the patent:
**06.05.2020 Bulletin 2020/19**

(21) Application number: **14762310.2**

(22) Date of filing: **14.03.2014**

(51) Int Cl.:
***A61M 1/00*** *(2006.01)*

(86) International application number:
**PCT/US2014/029319**

(87) International publication number:
**WO 2014/144769 (18.09.2014 Gazette 2014/38)**

(54) **METHODS AND COMPUTER SYSTEM FOR DETERMINING A PATIENT PARAMETER, INCLUDING A VOLUME OF FLUID DRAINAGE**

VERFAHREN UND COMPUTERSYSTEM ZUR BESTIMMUNG EINES PATIENTENPARAMETERS UMFASSEND DAS VOLUMEN EINER DRAINAGEFLÜSSIGKEIT

PROCÉDÉS ET SYSTÈME D'ORDINATEUR POUR DÉTERMINER UN PARAMÈTRE DE PATIENT COMPRENANT UN VOLUME DE DRAINAGE D'UN FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361794712 P**

(43) Date of publication of application:
**20.01.2016 Bulletin 2016/03**

(73) Proprietor: **Atrium Medical Corporation Merrimack, NH 03054 (US)**

(72) Inventors:
• **PEATFIELD, Greg**
  **Atkinson, New Hampshire 03811 (US)**

• **KAROWSKI, Theodore**
  **Hollis, New Hampshire 03049 (US)**

(74) Representative: **Zacco GmbH Bayerstrasse 83 80335 München (DE)**

(56) References cited:
**WO-A1-2013/003970    US-A1- 2005 154 359**
**US-A1- 2007 019 865    US-A1- 2010 280 334**
**US-A1- 2011 071 415    US-A1- 2011 071 415**
**US-A1- 2012 059 340**

**Description**

RELATED APPLICATIONS

This application claims priority to a United States provisional patent application Serial Number 61/794,712 filed March 15, 2013, and United States non-provisional patent application Serial Number 14/212,336 filed March 14, 2104.

FIELD OF THE INVENTION

Embodiments of the present invention are directed to a computer system and methods in which it is desired to know the amount of fluid removed from a source of fluid such as the chest cavity of an individual.

BACKGROUND

There are several disease states and injuries for which it is desirable to remove fluids from the chest cavity of the afflicted individual. Clinicians monitor the amount of fluid removed in order to determine the progression of the healing process, the progression of the disease and the function of the devices used to effect such drainage. Typically, the patient has a drainage tube inserted into the chest cavity and the tube drains into a special closed container known as a chest drain unit (CDU). There are various means to measure the fluid in the sealed container and each is associated with a cost, either in clinician time in making an observation or measurement, or in equipment cost in providing sensors and signaling devices for making a measurement and bringing the measurement data to the attention of the clinician or a hospital information system.

For example, without limitation, some devices feature one or more strain gauges for measuring the weight or changing weight of a container. As the accuracy of the strain gauge increases, the gauge becomes more expensive and more susceptible to damage. Strain gauges in particular are easily damaged, and if designed to be more robust have less resolution to weight change. US 2011/0071415 A1 discloses a drainage system and method of use. WO 2013/003970 A1 discloses a drainage device having reduced counter-pressure. US 2012/0059340 A1 discloses another drainage apparatus and method.

Clinical staff monitoring of a patient on a CDU, required to record fluid drainage volume levels of a patient, is awkward, time consuming and, therefore, costly and not measured or checked as often as otherwise might be desirable. The drainage devices are usually kept on the floor and require a clinician to get down on hands and knees to read the fluid levels, and remember to adjust for errors should for instance the CDU accidentally be knocked over during use.

SUMMARY OF THE INVENTION

Embodiments of the present invention are directed methods and computer system for determining at least one patient parameter, in a patient having the drain tube having a first end and a second end, the first end fitted into the patient chest cavity and a second end received in a collection vessel. As used herein, the term "patient parameter" refers to a patient parameter associated with the drain tube, including, by way of example, without limitation, volume of fluid (gas and/or liquid) removed from the chest cavity, the flow rate of the fluid being removed, the present volume of the container holding the liquid portion of the removed fluid, present function of filtering devices and tubes, and patient breathing status.

Embodiments of the invention feature at least one of the collection vessel and a drain tube having a first pressure sensor sensing the pressure within at least one of (i) the drain tube in a location proximal to the collection vessel and (ii) the collection vessel, and a second pressure sensor sensing the pressure within the drain tube at a location distal to the collection vessel. That is, the first pressure sensor is closely associated with the collection vessel either located within the collection vessel or in the drainage tube close to the collection vessel. The second pressure sensor is positioned in the drainage tube away from the collection vessel, more proximal to the patient.

The method comprises the steps of obtaining at least a first pressure value from the first pressure sensor and at least a first pressure value from the second pressure sensor. The method further comprises the step calculating at least one patient parameter by the pressure values through a non-linear solver.

One embodiment of the present method features associating each pressure value with a time and obtaining a plurality of successive pressure values over time. The successive pressure values are preferably separated in time at timed intervals; that is, it is periodic.

Embodiments of the present method are well suited for performance by computer means. As used herein, the term "computer means" refers to computer or central processing units (CPUs), internal to a device or carried separate and distinct from a device, such as CPUs carried on mainframe computers, servers, conventional desktop and laptop computers, microcontrollers, board computers such as Raspberry Pi, and handheld communication devices such as cell phones and tablet devices, off-site computers linked through internet and/or wireless communication devices and the like. Preferably, the first pressure values and successive pressure values are stored in computer memory. As used herein, the term "computer memory" refers to data storage such as chips, disks, memory drives, hard drives, flash drives and the like that are in signal communication with the CPU and can be processed by such CPU. For example, the CPU accesses such first in time pressure values and the successive pressure values from memory and performs the step of organizing such data, calculating at least one patient parameter and produces an output displaying the one patient parameter.

As used herein, the term "non-linear solver" refers to a machine learning process in a hypothesis/model mathematical computer program sense. The non-linear solver is trained with known inputs and outputs and the trained non-linear solver is exported or programed to receive the first pressure value and second pressure value and all other successive pressure values, process such values through the hypothesis/model to obtain a patient parameter,.

Embodiments feature a non-linear solver implemented in one or more approaches selected from the group consisting of Support Vector Machine Approach, Artificial Neural Networks, Genetic Algorithms and Genetic Programming. The detailed discussion will describe a non-linear equation in the form of a linear regression hypothesis and machine learning processes.

For example, one non-linear equation is the linear regression hypothesis:

$$h(T) = f\big(P_c(t), P_v(t)\big) = Theta_{bias} + \sum_{j=0}^{N} \big[Theta_{c,j} * P_C(j) + Theta_{V,j} * P_V(j)\big]$$

As used above:

T = Represent current time (t=0), or point at fixed time after data
t = Integer time sample, 0 most current, N - oldest
f(x,y) = An unknown function dependent upon x & y
h(T) = Volume of fluid in fluid canister
$P_V(t)$ = Pressure as a function of time at Vent,
$P_C(t)$= Pressure at CDU side of patient as a function of time,
N = Number of time samples required for calculation
Theta= Set of all Theta's listed below
$Theta_{bias}$= Arbitrary DC bias constant for hypothesis
$Theta_{c,t}$= Arbitrary parameter coefficient for CDU Pressure at time t
$Theta_{V,t}$= Arbitrary parameter coefficient for Vent Pressure at time t.

The linear regression equation has a cost function J expressed with training set of "m" values, expressed as follows:

$$J(Theta) = \frac{1}{2*m} \sum_{i=1}^{m} \big[h(T)^i - V(T)^i\big]^2$$

The unknown "Theta" values are determined with training set of 'm' items as follows:

$$\min_{Theta} J(Theta) = \frac{1}{2*m} \sum_{i=1}^{m} \big[h(T)^i - V(T)^i\big]^2$$

For example, without limitation, the non-linear equation and its derivatives are solved by one or more optimization algorithms chosen from the group consisting of Gradient Descent, Particle Swarm Optimization and Artificial Bee Colony.

The one method features *Theta* derived as follows:

$$Theta_x = Theta_x - \alpha * \frac{\partial}{\partial Theta_x} J(Theta)$$

with the partial derivative for J(Theta) with $X_x$ being the Bias, Pv or Pc parameter associated with $Theta_x$ expressed as:

$$\frac{\partial}{\partial Theta_x}J(Theta) = \frac{1}{m}\sum_{i=1}^{m}\left[h_{Theta}(T)^i - V_{Theta}(T)^i\right] * X_x^i.$$

A further non-linear equation embodying features of the present invention is expressed as:

$$h(T) = f\big(P_c(t),P_v(t)\big)$$
$$= Theta_{bias}$$
$$+ \sum_{j=0}^{N}\left[Theta_{C1,j} * P_C(j) + Theta_{C2,j} * P_C(j)^2 + Theta_{V1,j} * P_V(j) + Theta_{V2,j} * P_V(j)^2\right]$$

wherein:

T = Represent current time (t=0), or point at fixed time after data
t = Integer time sample, 0 most current, N - oldest
f(x,y) = An unknown function dependent upon x & y
h(T) = Volume of fluid in fluid canister
$P_V(t)$ = Pressure as a function of time at Vent,
$P_C(t)$= Pressure at CDU side of patient as a function of time,
N = Number of time samples required for calculation
Theta= Set of all Theta's listed below
$Theta_{bias}$= Arbitrary DC bias constant for hypothesis
$Theta_{c,t}$= Arbitrary parameter coefficient for CDU Pressure at time t
$Theta_{V,t}$= Arbitrary parameter coefficient for Vent Pressure at time t.

A further embodiment is directed to computer readable program for determining at least one patient parameter. The computer readable program comprises instructions for a computer processing unit having memory to obtain at least one first pressure value from the first pressure sensor and at least one first pressure value from the second pressure sensor. The computer readable program further instructs computer means to calculate at least one patient parameter by the pressure values through a non-linear solver.

The computer readable program operates or runs on computer means, to receive the at least one first pressure values and any successive pressure values over time, for example, at timed intervals, and store such data in memory. The data is organized and used to calculate one or more patient parameters for display. It is processed in accordance with the non-linear equations and methods described above.

A further embodiment is directed to a device for determining at least one patient parameter. The device comprises a first pressure sensor constructed and arranged to sense the pressure within at least one of (i) the drain tube in a location proximal to the collection vessel or (ii) the collection vessel itself, and a second pressure sensor constructed and arranged to sense the pressure within the drain tube at a location distal to the collection vessel. The first pressure sensor and the second pressure sensor are constructed and arranged for being placed in signal communication with computing means having memory for obtaining a first pressure value from the first pressure sensor and a first pressure value from the second pressure sensor. The computer means calculates at least one patient parameter by the pressure values through a non-linear solver.

A preferred device comprises a computer means. The computer means preferably receives a plurality of first pressure values and any successive pressure values over time, for example, without limitation, at timed intervals, which are stored in memory, organized and processed to calculate one patient parameter.

The data is organized and used to calculate one or more patient parameters for display. It is processed in accordance with the non-linear equations and methods described above and described more fully in the detailed description that follows.

The methods, computer readable programs and devices allow monitoring of fluids removed from the chest cavity of a patient. Such fluids comprise any biological fluid such as gas, water, serum, blood, plasma, lymph, pus therapeutic infusion fluids and mixtures thereof. The term "gas" is used to refer to air and gases administered for therapeutic purposes, such as, oxygen, nitrogen, helium and nitric oxide. Embodiments of the present method computer readable program and device allow the calculation of the gas volume and the liquid volume and corresponding flow rate of the fluid.

These and other features and advantages will be apparent to those skilled in the art upon viewing the Figures described briefly below and studying the detailed description of the invention that follows.

BRIEF DESCRIPTION OF THE FIGURES

FIG. 1 shows one exemplary fluid analyzer for determining volume and/or flow of fluid draining from a chest cavity of a patient, according to an example embodiment.

FIG. 2 is a schematic showing the electronic control module of FIG. 1 in further exemplary detail, according to an example embodiment.

FIG. 3 is a flowchart illustrating one exemplary method for collecting and storing time series pressure values, according to an example embodiment.

FIG. 4 is a flowchart illustrating one exemplary method for processing the stored time series pressure values of FIG. 3 for measurement of chest drain fluid, according to an example embodiment.

FIG. 5 is a schematic illustrating one exemplary electronic circuit that models the drainage device of FIG. 1, according to an example embodiment.

FIG. 6 depicts a machine learning process, according to an example embodiment.

DETAILED DESCRIPTION OF THE EMBODIMENTS

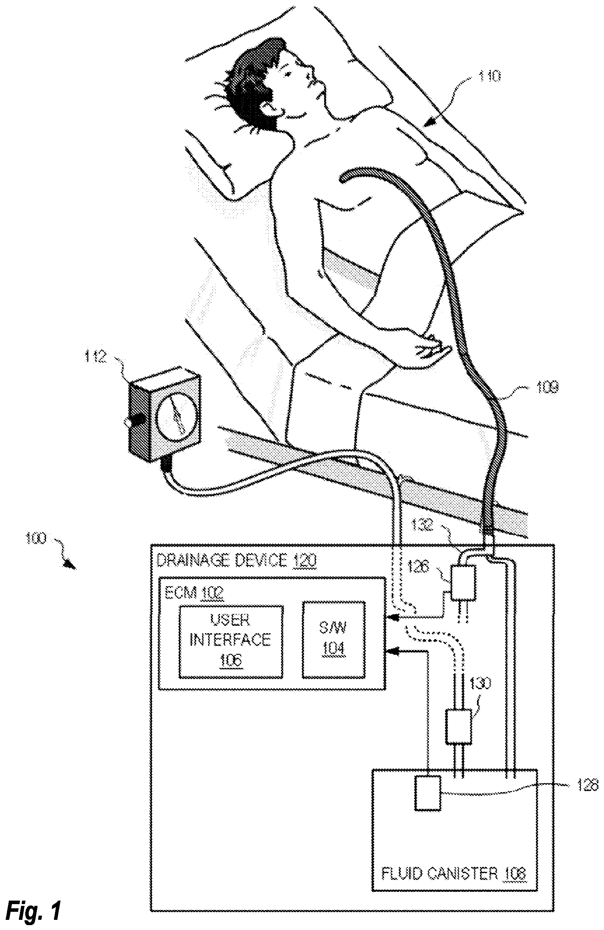

Embodiments will be described in detail with respect to a device, system and method for measuring fluid volumes from an external source such as a chest cavity of a patient. Turning now to FIG. 1 a device embodying features of the present invention, sometimes referred herein as fluid analyzer, and generally designated by the numeral 100, is depicted.

FIG. 1 shows one exemplary fluid analyzer 100 for determining volume and/or flow of fluid draining from source such as a chest cavity of a patient 110. In the example of FIG. 1, fluid analyzer 100 is implemented within a drainage device 120 that operates, for example, to collect fluid drained from a chest cavity of a patient 110 using a catheter or tubing such as a double-lumen catheter 109. In the example of FIG. 1, drainage device 120 is shown coupled to a vacuum source 112 to facilitate fluid drainage from patient 110.

Drainage device 120 is shown to include an electronic control module (ECM) 102 that operates to control drainage of fluid through catheter 109 into a fluid canister 108. As depicted, ECM 102 includes a user interface 106 for interacting with a user (e.g., clinical staff) of device 120. Fluid canister 108 is for example a chest drainage unit (CDU) that collects fluid draining from a chest cavity of a patient. ECM 102 receives pressure values from (a) a vent pressure sensor 126 that senses pressure near the patient connection of a vent pathway of catheter 109 and (b) a CDU pressure sensor 128 that senses pressure at the end of a drain tube and collection system that originates from the same patient point. Vent pressure sensor 126 may be located on the drain pipe of catheter 109, particularly where catheter 109 is not of a double lumen type. Additional pressure sensors, positioned with drainage device 120 and/or catheter 109, may be used with system 100 without departing from the scope hereof.

Drainage device 120 may include other components (e.g., valves, filters, sensors, converts, interfaces, etc.) without departing from the scope hereof. Drainage device 120 processes a time series of pressure values from pressure sensors 126 and 128 to determine fluid volume and/or flow rate of fluid drained from patient 110. ECM 102 may determine other metrics such as, but not limited to: CDU filter clogging, patient breathing, patient inhaling, patient exhaling, breaths/min, patient in respiratory distress (e.g., coughing, choking, laughing, crying, etc.), tube clogs and the degree of occlusion or clogging, fluid in line, and patient dumping of fluid.

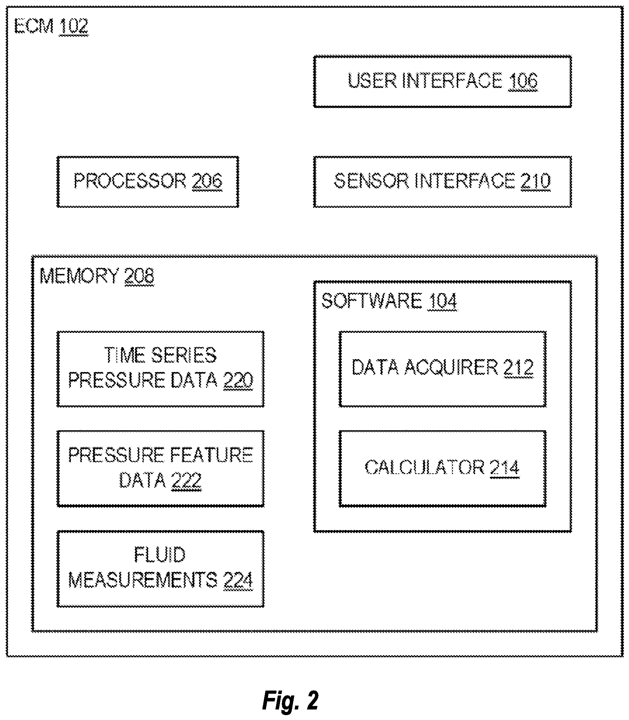

FIG. 2 is a schematic showing ECM 102 in further exemplary detail. FIGs. 1 and 2 are best viewed together with the following description. ECM 102 is shown to include a user interface 106, a processor 206, a memory 208, and a sensor interface 210. Memory 208 is illustratively shown storing software 104 that includes machine readable instructions that when executed by processor 206 implements functionality within drainage device 120 for determining a volume and/or flow rate of the fluid draining from patient 110. Sensor interface 210 couples with both vent pressure sensor 126 and CDU pressure sensor 128 and includes an analog to digital converter to provide sensed pressure values to software 104. Software 104 includes a data acquirer 212 and a calculator 214. Data acquirer 212 operates to collect pressure values from pressure sensors 126 and 128 over time and stores these values as time series pressure data 220 within memory 208. Time series pressure data 220 contains pressure values read periodically from pressure sensors 126 and 128 via sensor interface 210. Calculator 214 operates to process time series pressure data 220 to determine volume of fluid within canister 108 and optionally determines a flow rate of fluid into canister 108. Calculator 214 may include a learning algorithm that automatically learns, based upon pressure values read from sensor interface 210 and defined volumes, a relationship between these pressure values and fluid volume and/or flow rate. Calculator 214 generates pressure feature data 222 based upon identified pressure variations at each pressure sensor 126 and 128. Pressure feature data 222 may be referred to hereinafter as vent pressure features and/or CDU pressure features. Calculator 214 also generates fluid measurements 224 to include one or both of fluid volume within canister 108 and fluid flow rate into canister 108. Data acquirer 212 and calculator 214 may operate concurrently.

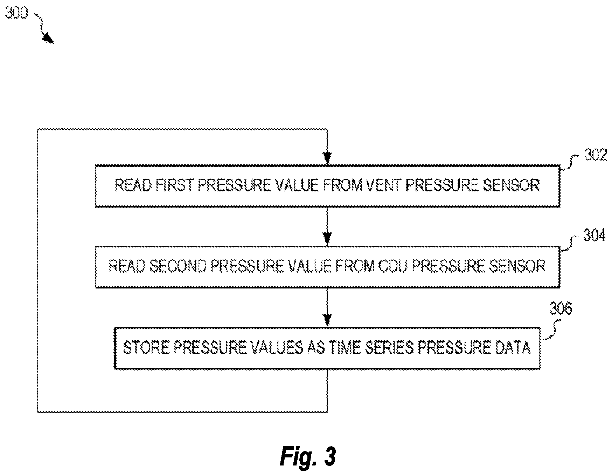

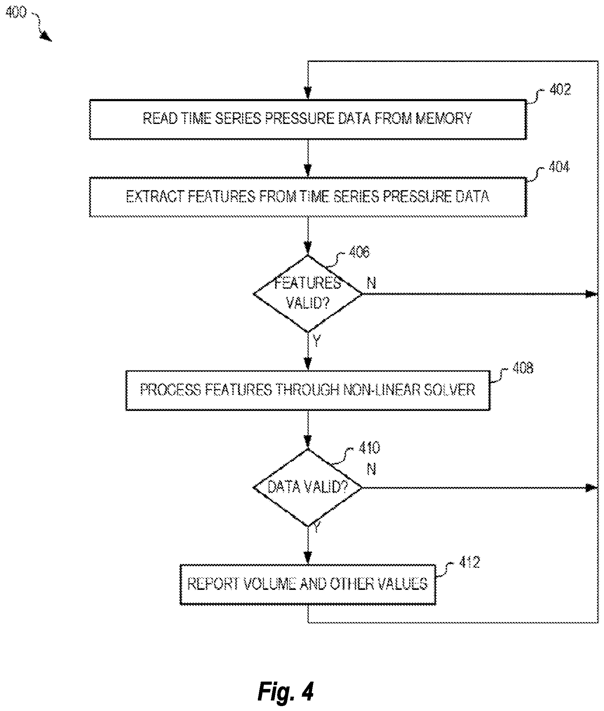

FIG. 3 is a flowchart illustrating one exemplary method 300 for reading and storing time series pressure values. FIG. 4 is a flowchart illustrating one exemplary method for processing the stored time series pressure data for measurement of chest drain fluid. Methods 300 and 400 are for example separate processing threads running on a computer is implemented within software 104 of ECM 102, for example. FIGs. 1 through 4 are best viewed together with the following description.

In step 302, method 300 reads a first pressure value from the vent pressure sensor. In one example of step 302, software 104, executed by processor 206, reads the first pressure value from vent pressure sensor 126 using sensor interface 210. In step 304, method 300 reads a second pressure value from the CDU pressure sensor. In one example of step 304, software 104, executed by processor 206, reads the second pressure value from CDU pressure sensor 128 using sensor interface 210.

In step 306, method 300 stores the pressure values of steps 302 and 304 as time series pressure data. In one example of step 306, data acquirer 212 stores pressure values of steps 302 and 304 within memory 208 as time series pressure data 220.

Method 300 repeats periodically to collect and store pressure values and calculates fluid parameters based upon the first and second pressure values.

In step 402, method 400 reads time series pressure data from memory. In one example of step 402, calculator 214 reads time series pressure data 220 from memory 208. In step 404, method 400 extracts features from the time series pressure data. In one example of step 404, calculator 214 extracts pressure features from time series pressure data 220 and stores these pressure features as pressure feature data 222 within memory 208.

Step 406 is a decision. In step 406, method 400 determines whether the features determined in step 404 are valid, and if these features are valid, method 400 continues with step 408; otherwise method 400 continues with step 402.

In step 408, method 400 processes the features through a non-linear solver. In one example of step 408, calculator 214 processes pressure feature data 222 through a non-linear equation to generate fluid measurements 224 that include one or both of fluid volume and fluid flow rate. In another example of step 408, calculator 214 processes pressure feature data 222 through a pre-trained Support Vector Machine. In another example of step 408, calculator 214 processes pressure feature data 222 using one or more of Artificial Neural Networks, Genetic Algorithms, and Genetic Programming. For example, calculator 214 may utilize data based upon one or more of collected training, validation and testing data sets.

Step 410 is a decision. In step 410, method 400 determines whether the data generated in step 408 is valid, and if this data is valid, method 400 continues with step 412; otherwise method 400 continues with step 402.

In step 412, method 400 reports the determined volume and other values. In one example of step 412, calculator 214 outputs fluid measurements 224 to user interface 106.

Method 400 repeats periodically to determine and output fluid measurements 224 based upon time series pressure data 220.

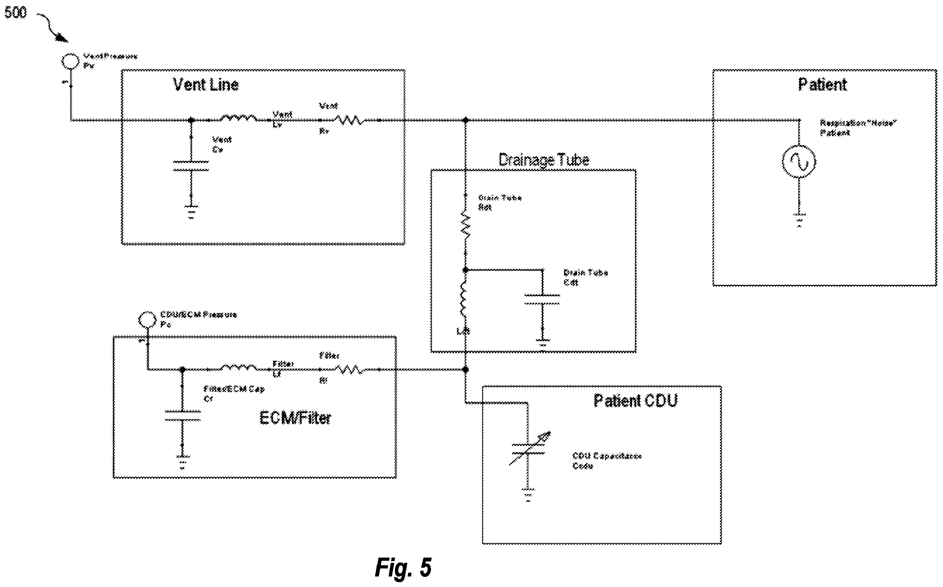

FIG. 5 shows one electronic analog 500 representing the resistance, inductance, and capacitance of different sections of drainage device 120. Thus, according to an example embodiment, electronic analog 500 models drainage device 120. Drainage device 120, however, could alternately be modeled in other manners without departing from the scope hereof. Electronic analog 500 is fixed for both branches, with the single exception of fluid canister 108, which is modeled by a variable capacitor. Given the dynamic pressure signal created by the patient and the "electrical network" differences "seen" by each pressure sensor 126, 128, this timing will vary non-linearly depending upon the fluid volume within fluid canister 108. An increase in fluid volume in fluid canister 108 will decrease the air capacity in the leg of the circuit affecting the pressure timing. The Filter (Rf of FIG. 5) could vary in resistance (increase over time) within the system. This resistance variation should be a linear affect and occur gradually over time.

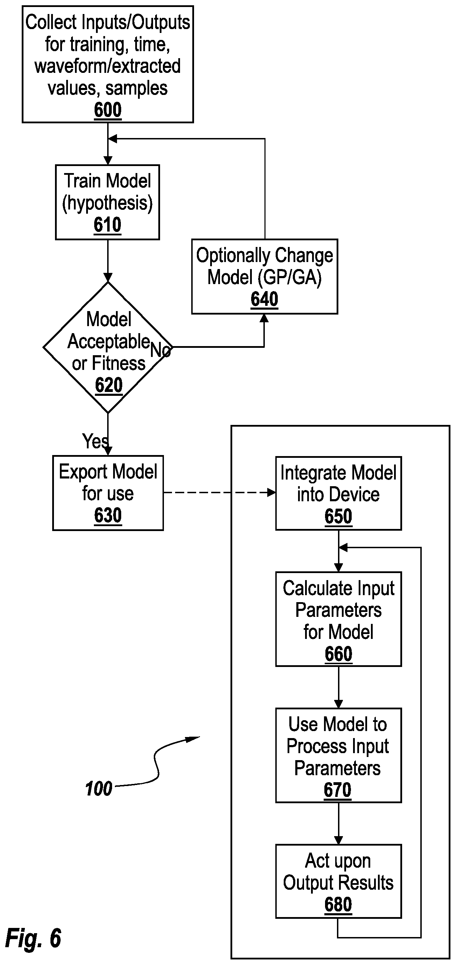

FIG. 6 depicts a machine learning process for a system or device 100. Steps 600, 610, 620 and 640 are characterized as training. The step 600 is collecting inputs and outputs for training. These inputs and outputs would be obtained experimentally in a controlled setting to produce a matrix of inputs and outputs in the training step 610. The training step 610 produces one or more outputs which are evaluated for acceptance or fitness. Genetic Algorithms and Programs evaluates inputs and outputs on the basis of fitness based upon desired outputs and upon all expected outputs during training compiled in to one value with optional changes to the model at step 640. If the model produces acceptable results, the model is exported at step 630 to device 100.

The model is integrated into the device 100 at step 650. And, the model is used to calculate input parameters for the model at step 660. The model is used to process the input parameters at step 670 and at step 680, the device 100 acts upon the output results.

**Exemplary Approach:**

In certain embodiments, a non-linear equation hypothesis is used to calculate the volume of fluid in fluid canister 108. Either a fixed model or dynamic and self-correcting model may be used. A fixed model is typically desired so that constants (weights or masses) do not change and can be used as-is within the software 104 without retraining. The hypothesis would be the same for either the fixed model or the dynamic and self-correcting model, but the equation may need to be optimized for the model.

**Hypothesis:**

LET:

- T = Represent current time (t=0), or point at fixed time after data
- t = Integer time sample, 0 most current, N - oldest
- f(x,y) = An unknown function dependent upon x & y
- J(Theta) = Cost of hypothesis vs actual for Theta
- V(T) = Volume of fluid in fluid canister 108 (actual)
- h(T) = Volume of fluid in fluid canister 108 (hypothesis)
- $P_V(t)$ = Pressure as a function of time at Vent, determined from pressure sensor 126, for example (Or vent pressure features, and other parameters)
- $P_C(t)$= Pressure at CDU side of patient as a function of time, determined from pressure sensor 128, for example (Or CDU pressure features, and other parameters)
- m = Number of samples in training set
- N = Number of time samples required for calculation
- Theta= Set of all Theta's listed below
- $Theta_{bias}$= Arbitrary DC bias constant for hypothesis
- $Theta_{c,t}$= Arbitrary parameter coefficient for CDU Pressure at time t
- $Theta_{V,t}$= Arbitrary parameter coefficient for Vent Pressure at time t

To approximate V(T), set the following Linear Regression hypothesis:

$$h(T) = f\big(P_c(t), P_v(t)\big) = Theta_{bias} + \sum_{j=0}^{N} \big[Theta_{c,j} * P_C(j) + Theta_{V,j} * P_V(j)\big]$$

A cost function J can be expressed as shown with training set of 'm' items as follows:

$$J(Theta) = \frac{1}{2*m} \sum_{i=1}^{m} \big[h(T)^i - V(T)^i\big]^2$$

Unknown "Theta" values can be found with training set of 'm' items as follows:

$$\min_{Theta} J(Theta) = \frac{1}{2*m} \sum_{i=1}^{m} \big[h(T)^i - V(T)^i\big]^2$$

There are a number of ways this equation may be solved or modified. One possible approach to solving or modifying the equation is through the use of a "Gradient Descent" approach/algorithm. Gradient Descent approaches provide a good approximation of solutions for the equation. Alternative methods include more recent and faster optimization systems, such as Particle Swarm Optimization systems. The equation is capable of being solved rapidly with low overhead using Artificial Bee Colony (ABC). Another approach is to use an external math package such as MatLab, R, or Octave, to solve for "Theta". These solution approaches are known in the art. These values of Theta are, for example, entered or otherwise incorporated into ECM 102, such as in the form of software 104. This approach is typically sufficient for any static "Theta" group.

Gradient Descent requires the partial derivative for the cost function in terms of "Theta" to move "Theta" downhill as follows:

$$Theta_x = Theta_x - \alpha * \frac{\partial}{\partial Theta_x} J(Theta)$$

The partial derivatives for J(Theta) with $X_x$ being the Bias, Pv or Pc parameter associated with $Theta_x$ can be expressed as:

$$\frac{\partial}{\partial Theta_x} J(Theta) = \frac{1}{m} \sum_{i=1}^{m} \left[ h_{Theta}(T)^i - V_{Theta}(T)^i \right] * X_x^i$$

If the above equation hypothesis does not converge, second power terms, and potentially even third power terms, may be added to help achieve convergence. Such incorporation of additional power terms will be required if the solution exhibits a high bias condition (under-fit). The opposite problem may occur if too many parameters are present, which is referred to high variance (over-fit). Over-fitting can be resolved through a number of techniques, such as regularization and adding more samples.

The following is second equation hypothesis including higher degree terms, which is used, for example, if first order model does not satisfactorily converge:

$$h(T) = f\left(P_c(t), P_v(t)\right)$$
$$= Theta_{bias}$$
$$+ \sum_{j=0}^{N} \left[ Theta_{C1,j} * P_C(j) + Theta_{C2,j} * P_C(j)^2 + Theta_{V1,j} * P_V(j) + Theta_{V2,j} * P_V(j)^2 \right]$$

Accuracy of the above mathematical models could be improved by use of derived parameters. For example, a maximum value maximum value and time difference between the two waveforms can be derived from the pressure waveforms. Using a derived parameter dataset rather than analysis over a large time series will reduce the "error" term in the cost calculation. Without parameter extraction of the raw data, many more samples will need to be processed to eliminate the error.

A more complex solution to the equation is through decomposition of the time signal into its Intrinsic Mode Functions or IMF. Intrinsic Mode Functions (IMF) can be used to improve accuracy at the expense of increased complexity/computation. Recent work has been done by Norden E. Huang in the form of the Hilbert-Huang Transform (HHT). This approach uses Empirical Mode Decomposition (EMD) and Hilbert Spectral Analysis (HAS) to improve accuracy, with drawback of increased computational expense. In a particular embodiment, the mathematical equation hypotheses discussed above are tested, and HHT techniques are implemented only if the mathematical hypotheses are unsatisfactory.

Drainage device 120 is capable of determining information in addition to volume of fluid in fluid canister 108 using, at least in part, pressure data from pressure sensors 126, 128. Additionally, certain embodiments further include one or more valve sensors (not shown) or programmed timing of the valve to detect the state of one or more valves in drainage device 120. In these embodiments, ECM 102 uses valve data from the valve sensors, along with data from pressure sensors 126, 128, for example, to determine additional information. ECM 102 determines this additional information, for example, using Linear Regression techniques, such as techniques similar to those discussed above for determining fluid canister 108 fluid volume. As another example, in some embodiments, ECM 102 determines additional information using Logistic Regression (also called Classification, probability output/Class present).

As discussed above, drainage device 120 is capable of estimating approximate volume of fluid in fluid canister 108. Accuracy of such an estimate is inversely proportional to the fluid volume due to changes in the system's "air capacity" in fluid canister 108. Additionally, certain embodiments are capable of detecting fine volume changes using information characterizing filter changes over time. Drainage device 120 determines filter changes, for example, by detecting change in resistance of a variable resistor, due to dampening of the filter. Furthermore, some embodiments are capable of detecting a heavily dumping patient 110, such as due to a broken stitch or suture, based on how fluid canister 108 volume may change over time.

Some embodiments are capable of determining air flow (also considered a fluid) through fluid canister 108 via a Liner Regression Model including, within drainage device 120, valve data from operation of a patient pressure relieving valve 130 and CDU pressure data from pressure sensor 128. Accuracy is increased, for example, by using feedback from the system and information characterizing change in current air passing. Moreover, some embodiments are capable of allowing for a tidal of air to enter fluid canister 108 and holding a valve to approximate the air pressure before release, to further promote accuracy. In these embodiments, drainage device 120 typically only holds the pressure for a split second to achieve better clinical data without significantly affecting therapy. Safety limits would be set around a feature such as this one. Pressure time decay data can be used to help approximate the volume/bolus passed to be summed over a time period to report air flow rate.

Respiration rate could be found using a similar approach but may require a large time data set (or samples from above data set).

Certain embodiments of drainage device 120 are capable of determining the existence, or probability of existence, of one or more of the following events using one or more Logistic Regression models:

- Volume Data from Linear Regression is valid

    ◦ Pressure signals not similar enough to training examples
    ◦ Possible error in reported Volume

- fluid canister 108 Filter Clogging, please replace CDU
- Patient is breathing
- Patient is inhaling
- Patient is exhaling
    ◦ Calculated breaths/min based upon above events
- Patient condition, such as Patient in repertory distress (e.g., coughing, choking, laughing, crying, etc.)
- Tube Clogged (e.g., Fully occluded)
- Degree of Tube Occlusion
- Fluid in Line
- Patient Dumping Fluid

Certain embodiments of the systems and methods disclosed herein achieve one or more of the following advantages:

- Better patient recovery time;
- A tube line clearing device (TLC) can be embedded as part of drainage device 120 to clear the drainage tube when needed, and to detect dependent loops, monitor fluid collection and rate, air flow and rate;
- TLC can be activated when there is a high degree of confidence there is a dependent loop and fluid collecting. This will extend battery life, with less disruption to therapy with un-needed TLC firing. There is less risk to patient when activating TLC with no clog.
- Patient safety is improved through better monitoring, e.g., the system can detect if a patient is connected properly to system and/or if the patient is breathing. Clinical help can be summoned in this extreme case.
- The system can determine if a drainage tube is occluded or has fluid blocking a path, fire TLC upon fluid blockage and alert clinical on kinked tube, and the like.
- The system provides more value in the data to record in patient record or for use in research or to send to central nursing stations
- Systems networked into Central Nursing Stations can provide the nursing staff with information for the following:

    ◦ Respiration Rate (Patient Tube Disconnected, Sleep or non-sleep related Apnea)
    ◦ Patient Distress
    ◦ Volume of Fluid
    ◦ Air Flow rate
    ◦ Tube patency
    ◦ Patient actively dumping fluid.

In certain embodiments, the methods, device and software of the present invention are implemented after capturing a training set of data and then a test set recorded from the ECM. The training set of data contains, for example, many samples at 10ml volume increments. High variance (interpolated samples may have higher than expected error) in the calculations is addressed with additional training samples as well as modifications to the cost function and/or parameter set.

In various exemplary embodiments, the sample data set may contain "bad" data at different times/volumes. "Bad" data would be considered measurements where the system model has been violated and no longer acts with sufficient predictive certainty. One "bad" data example is data acquired when fluid is trapped in the drainage line or a clamp installed on the line. Additional "bad" examples might include sudden changes in respiration such as a cough and no respiration. Another example might include an air leak at different volumes.

One of skill in the art will recognize the availability of a variety of tools such as *Matlab,* and a free tool called *Octave,* to be used to analyze the data to find "theta" and then solve for volume and other parameters, as required.

## LOGISTIC REGRESSION

In certain embodiments, logistic regression is applied to develop a system which generates a classification of the inputs (e.g., Yes/No for clogged status, data valid/data not valid, patient breathing, etc.). Logistic regression is very similar to the above Linear Regression, with the equation hypothesis wrapped by the Sigmoid Function whose output is from 0 to 1.

$$sigmoid(x) = \frac{1}{1 + e^{-x}}$$

Expressed as a logistic hypothesis, the equation can take the form of:

$$Logistic\_h(T) = sigmoid\big(h(T)\big) = \frac{1}{1 + e^{-h(T)}}$$

The cost function is changed to keep a convex solution to help avoid local minima. The new form of the cost function where y is a 1 if it belongs to the class and 0 if it does not:

$$J(Theta) = \frac{-1}{m} \sum_{i=1}^{m} \left[ y^i * \log(h_{Theta}(x^i)) + (1 - y^i) * \log(1 - h_{Theta}(x^i)) \right]$$

The partial derivative is similar only in terms of the classifier y (0 or 1):

$$\frac{\partial}{\partial Theta_x} J(Theta) = \frac{1}{m} \sum_{i=1}^{m} \left[ h_{Theta}(T)^i - y_{Theta}(T)^i \right] * X_x^i$$

Classifications are done, for example, in an *One vs All* structure. That is, for each classification, the neural network would be trained such that only the class being trained is "in the class" or 1. All other samples will be "out of the class" or otherwise set for 0. This means that there will be a different "theta" set for each classification desired.

### Support Vector Machine Approach

Support vector machines are well suited and specialized in classification. By splitting all the above Logistic regression into a trained Support Vector Machine, (SVM) one gets a computationally efficient system generating a probability of a class being present in the input data.

SVMs differ from Logistic Regression in that a SVM "stores" the positive examples within its hyperplane (n-dimensional space). It then reports how close a sample is to one of the learned examples. The kernel selection is useful and defines the probability of the sample in relation to a known positive in the hyperplane. An open-source and optimized C++ library called "libSVM" may be used to prototype and/or be integrated into drainage device 120 for class prediction, according to a variety of example embodiments.

For the machine learning system to generalize to conditions that were not taught, the features themselves need to be general. For example, using the absolute pressure magnitude of both pressure sensors 126, 128 may not generalize well. The pressure applied to patient 110 and by the patient will be related to (i) the prescribed negative pressure and the force applied by the patient's respiratory system, and (ii) amount of air dead volume in the patient's pleural space.

The data received from pressure sensors 126, 128 is dependent upon the fluid canister 108 fluid volume, and features that are more generic can be formed. The following is a partial list of features that may be used in certain embodiments:

- Peak Delay (e.g., time in units of microseconds or milliseconds) - Using the slowest pressure sensor waveform (most likely fluid canister 108 pressure), report the time from its peak pressure back to the peak pressure of the vent pressure peak.
- Period Max-Min (pressure) for Vent
- Distance between Vent Pressure peaks (in units of time such as milliseconds)
- Period Max-Min (pressure) for fluid canister 108
- Distance between fluid canister 108 Pressure peaks (in units of time such as milliseconds)
- Pressure delta between fluid canister 108 & Vent at Vent Pressure Peak
- Pressure delta between fluid canister 108 & Vent at CDU Pressure Peak
- Slope of Vent pressure (leading to peak)
- Slope of fluid canister 108 pressure (leading to peak)
- Area under Pressure Curves
- Valve Timing (Open/Closed times)

One embodiment of the present invention features Machine Learning through Genetic Programming (GP). Unlike the Genetic Algorithm and Machine Learning approaches which optimize an assumed solution, GP creates a "program" to solve the problem. The result of the learning process is a "formula/source code" which solves the applied problem.

There are several different approaches to Genetic Programming, including the following:

- GP - Standard Genetic Programming based upon a functional tree of instructions
- LGP - Linear Genetic Programming, fixed instruction length linearly processed (Fast)
- CGP - Cartesian Genetic Programming, Fixed grid of random instruction points randomly wired

An example of a commercially available LGP application which can generate C code to easily embed within the software 104 is Discipulus 5 Genetic Programming Predictive Modeling (RML Technologies, Inc., Littleton, Colorado, http://www.rmltech.com/).

In certain embodiments, the vent pressure sensor 126 may be omitted. This may occur for instance if characteristics of an externally applied stimulus are known. Volume and other features may still be determined using the CDU pressure sensor 128 in fluid canister 108 based upon the known patient source. For example, where a patient is on a heart-lung machine or ventilator, such external stimulus is known.

Changes may be made in the above methods and systems without departing from the scope hereof. It should thus be noted that the matter contained in the above description or shown in the accompanying drawings should be interpreted as illustrative and not in a limiting sense. The present invention is defined by the scope of the claims.

**Claims**

1. A method for determining at least two patient parameters in a patient (110) having a drain tube (109) having a first end and a second end, said first end fitted into the patient chest cavity and a second end in fluid communication with a collection vessel (108), at least one of said collection vessel (108) and said drain tube (109) having a first pressure sensor (128) sensing the pressure within at least one of (i) said drain tube (109) in a location proximal to the collection vessel (108), and (ii) the collection vessel (108), and a second pressure sensor (126) sensing the pressure within the drain tube (109) at a location distal to the collection vessel (108),
wherein the determined patient parameters include an estimation of the volume of liquid fluid collected in the collection vessel (108) and drain tube clogging,
said method performed by computer means (206) and comprising the steps of:

   a. obtaining at least a first pressure value from said first pressure sensor (128) and at least one second pressure value from said second pressure sensor (126) and successive pressure values from said first pressure sensor and said second pressure sensor;
   b. storing the pressure values as time series pressure data in computer memory (208);
   c. reading the time series pressure data from the computer memory (208);

d. extracting pressure features from the time series pressure data;

e. calculating the estimation of the volume of liquid fluid collected in the collection vessel (108) by processing the extracted pressure features through a non-linear solver using a non-linear equation;

f. determining whether the drain tube (109) is clogged; and

d. if the drain tube (109) is clogged, activating a tube line clearance device in order to clear the drain tube.

2. The method of claim 1, wherein said pressure values are obtained at timed intervals.

3. The method of claim 2, wherein said pressure values are associated with one or more events, status or values selected from the group consisting of valve status, time, patient inhalation status, and tube status.

4. The method of any of the preceding claims, wherein said computer means (206) produces an output displaying said at least two patient parameters.

5. The method of any one of the previous claims, wherein said non-linear equation is implemented in one or more approaches selected from the group consisting of Support Vector Machine Approach, Artificial Neural Networks, Genetic Algorithms and Genetic Programming.

6. The method of any one of the previous claims, wherein said non-linear equation is the linear regression hypothesis:

$$h(T) = f\big(P_c(t), P_v(t)\big) = Theta_{bias} + \sum_{j=0}^{N}\big[Theta_{c,j} * P_C(j) + Theta_{V,j} * P_V(j)\big]$$

T = Represent current time (t=0), or point at fixed time after data

t = Integer time sample, 0 most current, N - oldest

f(x,y) = An unknown function dependent upon x & y

h(T) = Volume of fluid in fluid canister

$P_V(t)$ = Pressure as a function of time at Vent,

$P_C(t)$= Pressure at CDU side of patient as a function of time,

N = Number of time samples required for calculation

Theta= Set of all Theta's listed below

$Theta_{bias}$= Arbitrary DC bias constant for hypothesis

$Theta_{c,t}$= Arbitrary parameter coefficient for CDU Pressure at time t

$Theta_{V,t}$= Arbitrary parameter coefficient for Vent Pressure at time t.

7. The method of claim 6, wherein said linear regression equation has a cost function J expressed with training set of "m" values, expressed as follows:

$$J(Theta) = \frac{1}{2 * m}\sum_{i=1}^{m}\big[h(T)^i - V(T)^i\big]^2$$

8. The method of claim 7, wherein unknown "Theta" values are determined with training set of 'm' items as follows:

$$\min_{Theta} J(Theta) = \frac{1}{2 * m}\sum_{i=1}^{m}\big[h(T)^i - V(T)^i\big]^2$$

9. The method of claim 8, wherein said non-linear equation and its derivatives are solved by one or more optimization algorithms chosen from the group consisting of Gradient Descent, Particle Swarm Optimization and Artificial Bee Colony.

**10.** The method of claim 7, wherein *Theta* is derived as follows:

$$Theta_x = Theta_x - \alpha * \frac{\partial}{\partial Theta_x} J(Theta)$$

with the partial derivative for J(Theta) with $X_x$ being the Bias, Pv or Pc parameter associated with $Theta_x$ expressed as:

$$\frac{\partial}{\partial Theta_x} J(Theta) = \frac{1}{m} \sum_{i=1}^{m} \left[ h_{Theta}(T)^i - V_{Theta}(T)^i \right] * X_x^i.$$

**11.** The method of any one of claims 1 to 5, wherein said non-linear equation is expressed as:

$$h(T) = f\big(P_c(t), P_v(t)\big)$$

$$= Theta_{bias}$$

$$+ \sum_{j=0}^{N} \left[ Theta_{C1,j} * P_C(j) + Theta_{C2,j} * P_C(j)^2 + Theta_{V1,j} * P_V(j) \right.$$

$$\left. + Theta_{V2,j} * P_V(j)^2 \right]$$

wherein:

T = Represent current time (t=0), or point at fixed time after data
t = Integer time sample, 0 most current, N - oldest
f(x,y) = An unknown function dependent upon x & y
h(T) = Volume of fluid in fluid canister
$P_V(t)$ = Pressure as a function of time at Vent,
$P_C(t)$= Pressure at CDU side of patient as a function of time,
N = Number of time samples required for calculation
Theta= Set of all Theta's listed below

$Theta_{bias}$= Arbitrary DC bias constant for hypothesis
$Theta_{c,t}$= Arbitrary parameter coefficient for CDU Pressure at time t
$Theta_{V,t}$= Arbitrary parameter coefficient for Vent Pressure at time t.

**12.** The method of any preceding claims, wherein said fluid is a biological fluid comprising any one of the group consisting of gas, water, serum, blood, plasma, lymph, pus, therapeutic infusion fluids and mixtures thereof.

**13.** The method of any one of the previous claims, wherein the computer means (206) monitors at least one additional patient parameter selected from the group consisting of error in volume data, error in flow data, filter clogging, breathing status, and excessive fluid movements.

**14.** The method of any one of the previous claims, wherein the non-linear solver is trained.

**15.** A computer system programmed to perform the method of any one of the preceding claims.

**Patentansprüche**

**1.** Verfahren zum Bestimmen von mindestens zwei Patientenparametern bei einem Patienten (110) mit einem Drainageschlauch (109), der ein erstes Ende und ein zweites Ende aufweist, wobei das erste Ende in die Brusthöhle des Patienten eingepasst ist und ein zweites Ende in Fluidverbindung mit einem Sammelbehälter (108) steht, wobei mindestens eines von dem Sammelbehälter (108) und dem Drainageschlauch (109) einen ersten Drucksensor (128), der den Druck in mindestens einem von (i) dem Drainageschlauch (109) an einer Stelle proximal zu dem Sammelbehälter (108) und (ii) dem Sammelbehälter (108) erfasst und einen zweiten Drucksensor (126) aufweist, der den Druck innerhalb des Drainageschlauchs (109) an einer Stelle distal zu dem Sammelbehälter (108) erfasst, wobei die bestimmten Patientenparameter eine Schätzung des Volumens des in dem Sammelbehälter (108) gesammelten flüssigen Fluids und ein Verstopfen des Drainageschlauchs beinhalten, wobei das Verfahren durch Computermittel (206) ausgeführt wird und die folgenden Schritte umfasst:

a. Erhalten von mindestens einem ersten Druckwert von dem ersten Drucksensor (128) und mindestens einem zweiten Druckwert von dem zweiten Drucksensor (126) und aufeinanderfolgenden Druckwerten von dem ersten Drucksensor und dem zweiten Drucksensor;
b. Speichern der Druckwerte als Zeitreihendruckdaten in einem Computerspeicher (208);
c. Lesen der Zeitreihendruckdaten aus dem Computerspeicher (208) ;
d. Extrahieren von Druckmerkmalen aus den Zeitreihendruckdaten;
e. Berechnen der Schätzung des Volumens des in dem Sammelbehälter (108) gesammelten flüssigen Fluids durch Verarbeiten der extrahierten Druckmerkmale durch einen nichtlinearen Löser unter Verwendung einer nichtlinearen Gleichung;
f. Bestimmen, ob der Drainageschlauch (109) verstopft ist; und
d. wenn der Drainageschlauch (109) verstopft ist, Aktivieren einer Schlauchleitungsfreigabevorrichtung, um den Drainageschlauch freizugeben.

2. Verfahren nach Anspruch 1, wobei die Druckwerte in Zeitintervallen erhalten werden.

3. Verfahren nach Anspruch 2, wobei die Druckwerte einem oder mehreren Ereignissen, Status oder Werten zugeordnet sind, die aus der Gruppe bestehend aus Ventilstatus, Zeit, Inhalationsstatus des Patienten und Schlauchstatus ausgewählt sind.

4. Verfahren nach einem der vorhergehenden Ansprüche, wobei das Computermittel (206) eine Ausgabe erzeugt, welche die mindestens zwei Patientenparameter anzeigt.

5. Verfahren nach einem der vorhergehenden Ansprüche, wobei die nichtlineare Gleichung in einem oder mehreren Ansätzen implementiert ist, die aus der Gruppe bestehend aus Support Vector Machine Approach, künstlichen neuronalen Netzen, genetischen Algorithmen und genetischer Programmierung ausgewählt sind.

6. Verfahren nach einem der vorhergehenden Ansprüche, wobei die nichtlineare Gleichung die lineare Regressionshypothese ist:

$$h(T) = f\big(P_c(t), P_v(t)\big) = Theta_{bias} + \sum_{j=0}^{N} \big[Theta_{c,j} * P_C(j) + Theta_{V,j} * P_V(j)\big]$$

T = Aktuelle Zeit (t=0) oder fester Zeitpunkt nach Daten
t = Ganzzahlige Zeitprobe, 0 aktuellste, N älteste
f(x,y) = Eine unbekannte Funktion in Abhängigkeit von x und y
h(T) = Fluidvolumen im Fluidbehälter
$P_V(t)$ = Druck in Abhängigkeit von der Zeit bei der Entlüftung,
$P_c(t)$ = Druck auf der CDU-Seite des Patienten in Abhängigkeit von der Zeit,
N = Anzahl der für die Berechnung erforderlichen Zeitproben
Theta = Satz aller nachstehend aufgelisteten Theta
$Theta_{bias}$ = Arbiträre DC-Bias-Konstante für die Hypothese
$Theta_{c,t}$ = Arbiträrer Parameterkoeffizient für den CDU-Druck zum Zeitpunkt t
Thetav,t = Arbiträrer Parameterkoeffizient für den Entlüftungsdruck zum Zeitpunkt t.

7. Verfahren nach Anspruch 6, wobei die lineare Regressionsgleichung eine Kostenfunktion J aufweist, ausgedrückt durch einen Trainingssatz von "m" Werten, ausgedrückt wie folgt:

$$J(Theta) = \frac{1}{2*m}\sum_{i=1}^{m}\left[h(T)^i - V(T)^i\right]^2$$

8. Verfahren nach Anspruch 7, wobei unbekannte "Theta"-Werte mit dem Trainingssatz von "m"-Elementen wie folgt bestimmt werden:

$$\min_{Theta} J(Theta) = \frac{1}{2*m}\sum_{i=1}^{m}\left[h(T)^i - V(T)^i\right]^2$$

9. Verfahren nach Anspruch 8, wobei die nichtlineare Gleichung und ihre Ableitungen durch einen oder mehrere Optimierungsalgorithmen gelöst werden, die aus der Gruppe bestehend aus Gradientenverfahren, Partikelschwarmoptimierung und künstlichem Bienenvolk ausgewählt sind.

10. Verfahren nach Anspruch 7, wobei *Theta* wie folgt abgeleitet wird:

$$Theta_x = Theta_x - \alpha * \frac{\partial}{\partial Theta_x}J(Theta)$$

mit der partiellen Ableitung für J(Theta), wobei $X_x$ der mit $Theta_x$ verbundene Bias-, Pv- oder Pc-Parameter ist, ausgedrückt als:

$$\frac{\partial}{\partial Theta_x}J(Theta) = \frac{1}{m}\sum_{i=1}^{m}\left[h_{Theta}(T)^i - V_{Theta}(T)^i\right] * X_x^i.$$

11. Verfahren nach einem der Ansprüche 1 bis 5, wobei die nichtlineare Gleichung ausgedrückt wird als:

$$
\begin{aligned}
h(T) &= f\left(P_c(t), P_v(t)\right) \\
&= Theta_{bias} \\
&+ \sum_{j=0}^{N}\left[Theta_{C1,j} * P_C(j) + Theta_{C2,j} * P_C(j)^2 + Theta_{V1,j} * P_V(j)\right. \\
&\left.+ Theta_{V2,j} * P_V(j)^2\right]
\end{aligned}
$$

wobei:

T = Aktuelle Zeit (t=0) oder fester Zeitpunkt nach Daten
t = Ganzzahlige Zeitprobe, 0 aktuellste, N älteste
f(x,y) = Eine unbekannte Funktion in Abhängigkeit von x und y
h(T) = Fluidvolumen im Fluidbehälter
$P_V(t)$ = Druck in Abhängigkeit von der Zeit bei der Entlüftung,
$P_c(t)$ = Druck auf der CDU-Seite des Patienten in Abhängigkeit von der Zeit,
N = Anzahl der für die Berechnung erforderlichen Zeitproben
Theta = Satz aller nachstehend aufgelisteten Theta
$Theta_{bias}$ = Arbiträre DC-Bias-Konstante für die Hypothese

Theta$_{c,t}$ = Arbiträrer Parameterkoeffizient für den CDU-Druck zum Zeitpunkt t

Thetav,t = Arbiträrer Parameterkoeffizient für den Entlüftungsdruck zum Zeitpunkt t.

12. Verfahren nach einem der vorhergehenden Ansprüche, wobei das Fluid ein biologisches Fluid ist, umfassend ein beliebiges aus der Gruppe bestehend aus Gas, Wasser, Serum, Blut, Plasma, Lymphe, Eiter, therapeutischen Infusionsfluiden und Gemischen davon.

13. Verfahren nach einem der vorhergehenden Ansprüche, wobei das Computermittel (206) mindestens einen zusätzlichen Patientenparameter überwacht, der aus der Gruppe bestehend aus Fehler bei Volumendaten, Fehler bei Durchflussdaten, Filterverstopfung, Atmungsstatus und übermäßigen Fluidbewegungen ausgewählt ist.

14. Verfahren nach einem der vorhergehenden Ansprüche, wobei der nichtlineare Löser trainiert wird.

15. Computersystem, programmiert, um das Verfahren nach einem der vorhergehenden Ansprüche auszuführen.

## Revendications

1. Procédé pour déterminer au moins deux paramètres de patient chez un patient (110) ayant un tube de drainage (109) ayant une première extrémité et une seconde extrémité, ladite première extrémité étant ajustée dans la cavité thoracique du patient et une seconde extrémité étant en communication fluidique avec un récipient collecteur (108), au moins l'un dudit récipient collecteur (108) et dudit tube de drainage (109) ayant un premier capteur de pression (128) détectant la pression à l'intérieur d'au moins l'un (i) dudit tube de drainage (109) à un emplacement à proximité du récipient collecteur (108), et (ii) du récipient collecteur (108), et un second capteur de pression (126) détectant la pression à l'intérieur du tube de drainage (109) à un emplacement distal du récipient collecteur (108), dans lequel les paramètres déterminés du patient comportent une estimation du volume de fluide liquide collecté dans le récipient collecteur (108) et le colmatage du tube de drainage, ledit procédé étant mis en œuvre par des moyens informatiques (206) et comprenant les étapes :

   a. d'obtention d'au moins une première valeur de pression en provenance dudit premier capteur de pression (128) et d'au moins une seconde valeur de pression en provenance dudit second capteur de pression (126) et des valeurs de pression successives en provenance dudit premier capteur de pression et dudit second capteur de pression ;
   b. de stockage des valeurs de pression en tant que données de pression de série temporelle dans la mémoire d'ordinateur (208) ;
   c. de lecture des données de pression de série temporelle dans la mémoire d'ordinateur (208) ;
   d. d'extraction de caractéristiques de pression à partir des données de pression de série temporelle ;
   e. de calcul de l'estimation du volume de fluide liquide collecté dans le récipient collecteur (108) en traitant les caractéristiques de pression extraites à travers un solutionneur non linéaire à l'aide d'une équation non linéaire ;
   f. du fait de déterminer si le tube de drainage (109) est bouché ; et
   d. si le tube de drainage (109) est bouché, d'activation d'un dispositif de dégagement de conduite de tube afin de dégager le tube de drainage.

2. Procédé selon la revendication 1, dans lequel lesdites valeurs de pression sont obtenues à des intervalles temporisés.

3. Procédé selon la revendication 2, dans lequel lesdites valeurs de pression sont associées à un ou à plusieurs événements, états ou valeurs sélectionnés dans le groupe constitué de l'état de la valve, du temps, de l'état d'inhalation du patient et de l'état du tube.

4. Procédé selon l'une quelconque des revendications précédentes, dans lequel ledit moyen d'ordinateur (206) produit une sortie affichant lesdits au moins deux paramètres de patient.

5. Procédé selon l'une quelconque des revendications précédentes, dans lequel ladite équation non linéaire est mise en œuvre dans une ou plusieurs approches choisies dans le groupe constitué d'une approche par machine à vecteur de support, de réseaux de neurones artificiels, d'algorithmes génétiques et d'une programmation génétique.

6. Procédé selon l'une quelconque des revendications précédentes, dans lequel ladite équation non linéaire est l'hypothèse de régression linéaire :

$$h(T) = f(P_c(t), P_v(t)) = Theta_{bias} + \sum_{j=0}^{N} \left[ Theta_{c,j} * P_C(j) + Theta_{v,j} * P_V(j) \right]$$

T = Représente l'heure actuelle (t = 0), ou un point à une heure fixe après les données

t = échantillon de temps entier, 0 le plus récent, N - le plus ancien

f(x, y) = Une fonction inconnue dépendante de x & y

h(T) = Volume de fluide dans la cartouche de fluide

$P_v(t)$ = pression en fonction du temps au trou d'aération,

$P_c(t)$= Pression du côté CDU du patient en fonction du temps,

N = Nombre d'échantillons de temps requis pour le calcul

Theta = Ensemble de tous les Thêta énumérés ci-dessous

$Theta_{bias}$ = Constante de polarisation CC arbitraire pour l'hypothèse

$Theta_{c,t}$= coefficient de paramètre arbitraire pour la pression CDU au temps t

$Theta_{v,t}$= coefficient de paramètre arbitraire pour la pression du trou d'aération au temps t.

**7.** Procédé selon la revendication 6, dans lequel ladite équation de régression linéaire a une fonction de coût J exprimée avec un ensemble d'apprentissage de « m » valeurs, exprimée comme suit :

$$J(Theta) = \frac{1}{2*m} \sum_{i=1}^{m} \left[ h(T)^i - V(T)^i \right]^2$$

**8.** Procédé selon la revendication 7, dans lequel des valeurs « Thêta » inconnues sont déterminées avec un ensemble d'apprentissage de « m » éléments, comme suit :

$$\min_{Theta} J(Theta) = \frac{1}{2*m} \sum_{i=1}^{m} \left[ h(T)^i - V(T)^i \right]^2$$

**9.** Procédé selon la revendication 8, dans lequel ladite équation non linéaire et ses dérivés sont résolus par un ou plusieurs algorithmes d'optimisation choisis dans le groupe constitué de la descente de gradient, de l'optimisation des essaims de particules et de la colonie d'abeilles artificielles.

**10.** Procédé selon la revendication 7, dans lequel *Theta* est dérivé comme suit :

$$Theta_x = Theta_x - \alpha * \frac{\partial}{\partial Theta_x} J(Theta)$$

avec la dérivée partielle pour J(Theta), $X_x$ étant le paramètre Bias, Pv ou Pc associé à $Theta_x$ exprimé comme:

$$\frac{\partial}{\partial Theta_x} J(Theta) = \frac{1}{m} \sum_{i=1}^{m} \left[ h_{Theta}(T)^i - V_{Theta}(T)^i \right] * X_x^i.$$

**11.** Procédé selon l'une quelconque des revendications 1 à 5, dans lequel ladite équation non linéaire est exprimée comme :

$$h(T) = f\left(P_c(t), P_v(t)\right)$$

$$= Theta_{bias}$$

$$+ \sum_{j=0}^{N} \left[ Theta_{C1,j} * P_C(j) + Theta_{C2,j} * P_C(j)^2 + Theta_{V1,j} * P_V(j) \right.$$

$$\left. + Theta_{V2,j} * P_V(j)^2 \right]$$

dans lequel :

T = Représente l'heure actuelle (t = 0), ou un point à une heure fixe après les données
t = échantillon de temps entier, 0 le plus récent, N - le plus ancien
f(x, y) = Une fonction inconnue dépendante de x & y
h(T) = Volume de fluide dans la cartouche de fluide
$P_v(t)$ = pression en fonction du temps au trou d'aération,
$P_c(t)$ = pression du côté CDU du patient en fonction du temps,
N = Nombre d'échantillons de temps requis pour le calcul
Theta = Ensemble de tous les Thêta listés ci-dessous
$Theta_{bias}$ = Constante de polarisation CC arbitraire pour l'hypothèse
$Theta_{c,t}$= coefficient de paramètre arbitraire pour la pression CDU au temps t
$Theta_{v,t}$= coefficient de paramètre arbitraire pour la pression du trou d'aération au temps t.

12. Procédé selon l'une quelconque des revendications précédentes, dans lequel ledit fluide est un fluide biologique comprenant l'un quelconque du groupe constitué du gaz, de l'eau, du sérum, du sang, du plasma, de la lymphe, du pus, des fluides de perfusion thérapeutique et de mélanges de ceux-ci.

13. Procédé selon l'une quelconque des revendications précédentes, dans lequel le moyen d'ordinateur (206) surveille au moins un paramètre de patient supplémentaire choisi dans le groupe constitué d'une erreur dans les données de volume, d'une erreur dans les données de débit, d'un colmatage du filtre, d'un état respiratoire et de mouvements de fluide excessifs.

14. Procédé selon l'une quelconque des revendications précédentes, dans lequel le solutionneur non linéaire est entraîné.

15. Système informatique programmé pour mettre en œuvre le procédé selon l'une quelconque des revendications précédentes.

**Fig. 1**

**Fig. 2**

300

302
READ FIRST PRESSURE VALUE FROM VENT PRESSURE SENSOR

304
READ SECOND PRESSURE VALUE FROM CDU PRESSURE SENSOR

306
STORE PRESSURE VALUES AS TIME SERIES PRESSURE DATA

*Fig. 3*

400

402 — READ TIME SERIES PRESSURE DATA FROM MEMORY

404 — EXTRACT FEATURES FROM TIME SERIES PRESSURE DATA

406 — FEATURES VALID? — N

Y

408 — PROCESS FEATURES THROUGH NON-LINEAR SOLVER

410 — DATA VALID? — N

Y

412 — REPORT VOLUME AND OTHER VALUES

*Fig. 4*

**Fig. 5**

Collect Inputs/Outputs
for training, time,
waveform/extracted
values, samples
**600**

Train Model
(hypothesis)
**610**

Optionally Change
Model (GP/GA)
**640**

Model
Acceptable
or Fitness
**620**

No

Yes

Export Model
for use
**630**

Integrate Model
into Device
**650**

Calculate Input
Parameters
for Model
**660**

Use Model to
Process Input
Parameters
**670**

Act upon
Output Results
**680**

*100*

*Fig. 6*

**EP 2 968 707 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61794712 B
- US 14212336 B
- US 20110071415 A1
- WO 2013003970 A1
- US 20120059340 A1